# EUROPEAN PATENT SPECIFICATION

(11) **EP 3 727 340 B1**
(45) Date of publication and mention of the grant of the patent: **21.08.2024**
(21) Application number: 18834178.8
(22) Date of filing: 19.12.2018
(51) Int. Cl.: A61K 9/14, A61K 31/485, A61P 25/04

(54) **PHARMACEUTICAL COMPOSITION CONTAINING CROSSLINKED ION EXCHANGE RESIN**
PHARMAZEUTISCHE ZUSAMMENSETZUNG ENTHALTEND QUERVERNETZTE IONENAUSTAUSCHERHARZE
COMPOSITION PHARMACEUTIQUE COMPRENANT DES RÉSINES ÉCHANGEUSES D'IONS RÉTICULÉES

(30) Priority: 22.12.2017 US 201762609696 P; 02.03.2018 US 201862637431 P
(43) Date of publication of application: 28.10.2020
(73) Proprietor: DDP Specialty Electronic Materials US, LLC, Wilmington, DE 19805 (US); DDP Specialty Electronic Materials US 8, LLC, Collegeville, PA 19426 (US)
(72) Inventor: TOCCE, Elizabeth, Midland, MI 48674 (US); GEHRIS, Amie, Collegeville, PA 19426 (US); SZEP, Silvia, Collegeville, PA 19426 (US)
(74) Representative: Houghton, Mark Phillip
(86) International application number: PCT/US2018/066506
(87) International publication number: WO 2019/126325

(56) References cited:
- EP-A2- 0 367 746
- EP-A2- 2 260 834
- CA-A- 656 643
- US-A1- 2005 265 955
- US-A1- 2006 115 529
- US-A1- 2013 071 476
- EVA ROBLEGG ET AL: "Reformulation of a codeine phosphate liquid controlled-release product", DRUG DEVELOPMENT AND INDUSTRIAL PHARMACY, vol. 36, no. 12, 5 December 2010 (2010-12-05), US, pages 1454 - 1462, XP055564490, ISSN: 0363-9045, DOI: 10.3109/03639045.2010.487870

## Description

Legitimate drugs are subject to abuse. One method of abusing a drug is to use readily available solvents, such as, for example, salt solutions, to extract the drug from a legitimate dosage form such as, for example, a collection of tablets or capsules. The resulting solution could then be injected or swallowed, thus providing a larger dose, delivered in a shorter time, than would be easily consumed by using the legitimate dosage form. It would be desirable to provide a dosage form that reduces the ability of the drug to be extracted from the dosage form.

US 9,125,948 describes a method for loading a drug onto ion exchange resin particles.

US 2006/0115529 discloses fast-melting tablets comprising ion exchange resins, having taste masking and sustained release properties.

It is desired to provide a composition that contains both a drug and an ion exchange resin, with the ion exchange resin designed to inhibit the extraction of the drug from the composition using readily available solvents such as salt solutions.

The following is a statement of the invention.

A first aspect of the present invention is a pharmaceutical composition comprising a collection of particles, wherein the particles comprise a drug and an ion exchange resin, wherein the ion exchange resin comprises 14% to 30% polymerized units of one or more multifunctional vinyl monomer, by weight based on the dry weight of the ion exchange resin.

A second aspect of the present invention is a pharmaceutical composition comprising a collection of particles, wherein the particles comprise a drug and an ion exchange resin according to the first aspect of the invention, wherein 90% or more by weight of the particles pass through a mesh screen having openings of 150 µm, and wherein 90% or more by weight of the particles are retained on a mesh screen having openings of 106 µm.

The following is a detailed description of the invention.

As used herein, the following terms have the designated definitions, unless the context clearly indicates otherwise.

Sugar alcohols are linear or branched C2 to C12 hydrocarbons having at least two hydroxyl groups, cyclic or heterocyclic C5 to C12 hydrocarbons having 2 or more hydroxyl groups. Examples of sugar alcohols are sorbitol, mannitol, polyglycitol, maltitol, lactitol, isomalt, erythritol, glycerin, polydextrose, fructose, maltose, xylitol, 1,3-dihydroxypropane inositol, and carbohydrates such as glucose and sucrose.

A "polymer," as used herein is a relatively large molecule made up of the reaction products of smaller chemical repeat units. As used herein, "polymer" is synonymous with "resin." Polymers may have structures that are linear, branched, star shaped, looped, hyperbranched, crosslinked, or a combination thereof; polymers may have a single type of repeat unit ("homopolymers") or they may have more than one type of repeat unit ("copolymers"). Copolymers may have the various types of repeat units arranged randomly, in sequence, in blocks, in other arrangements, or in any mixture or combination thereof.

Molecules that can react with each other to form the repeat units of a polymer are known herein as "monomers." The repeat units so formed are known herein as "polymerized units" of the monomer.

Vinyl monomers have the structure 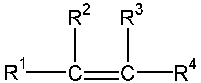 where each of R¹, R², R³, and R⁴ is, independently, a hydrogen, a halogen, an aliphatic group (such as, for example, an alkyl group), a substituted aliphatic group, an aryl group, a substituted aryl group, another substituted or unsubstituted organic group, or any combination thereof. Vinyl monomers are capable of free radical polymerization to form polymers. Some vinyl monomers have one or more polymerizable carbon-carbon double bonds incorporated into one or more of R¹, R², R³, and R⁴; such vinyl monomers are known herein as multifunctional vinyl monomers. Vinyl monomers with exactly one polymerizable carbon-carbon double bond are known herein as monofunctional vinyl monomers.

Acrylic monomers include, acrylic acid, methacrylic acid, unsubstituted alkyl esters thereof, substituted-alkyl esters thereof, unsubstituted amides thereof, N-substituted amides thereof, acrylonitrile, and methacrylonitrile. Substituents may be alkyl groups, hydroxyl groups, groups containing carbon-carbon double bonds, other organic groups, or combinations thereof. The prefix "(meth)acryl-" means either "acryl-" or "methacryl-".

Styrenic monomers are vinyl monomers in which each of R¹ and R² is hydrogen, R³ is hydrogen or alkyl, and -R⁴ has the structure 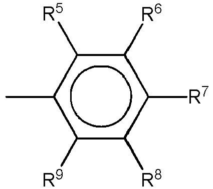 where each of R⁵, R⁶, R⁷, R⁸, and R⁹ is, independently, a hydrogen, a halogen, an aliphatic group (such as, for example, an alkyl group or a vinyl group), a substituted aliphatic group, an aryl group, a substituted aryl group, another substituted or unsubstituted organic group, or any combination thereof.

A reaction among monomers to form one or more polymers is referred to herein as a polymerization process.

A polymer is said herein to contain polymerized units of the monomers used in making the polymer, even if some or all of those polymerized units are, after polymerization, altered by the addition of one or more functional groups. For example, a copolymer made from styrene and divinylbenzene (DVB) in a weight ratio of styrene:DVB of 90: 10 is said to have 90% by weight polymerized units of styrene. If that copolymer were to be then altered, for example by reaction with sulfuric acid to replace some of the hydrogen atoms on aromatic rings in the polymerized units of styrene with sulfonic acid groups, the resulting functionalized polymer would still be said to have 90% by weight polymerized units of styrene.

Macroporous polymeric beads have a porous structure with average pore diameter of 20 nm or larger. Pore diameter is measured using the Brunauer-Emmett-Teller (BET) method using nitrogen gas. Macroporous polymeric beads are normally made by incorporating a porogen into monomer droplets. The porogen is soluble in the monomer, but the polymer does not dissolve in the porogen, so that, as the polymer forms, phase-separated domains of porogen remain. After polymerization, the porogen is removed by evaporation or by washing with solvent. The porous structure of the polymeric bead is the empty space left when the porogen is removed from its phase-separated domains.

Gel type polymeric beads are made without the use of porogen. The pores in gel type polymeric beads are the free volumes between the atoms in the entangled, possibly crosslinked polymer chains of the polymeric bead. The pores in gel type polymeric beads are smaller than 20 nm. In some cases, the pores in gel type resins are too small to be detected using the BET method.

As used herein, ion exchange is a process in which a solution comes into contact with an ion exchange resin. Prior to the contact with the solution, the ion exchange resin has functional groups of a certain charge that are covalently attached to the resin, and has ions of the opposite charge associated with the functional groups. When the solution comes in contact with the ion exchange resin, some ions in solution become attached to the ion exchange resin by exchanging places with ions of the same charge that had been associated with the functional groups on the ion exchange resin.

Some compounds have covalently attached cationic groups. A group is cationic if, when the compound is in contact with water, there is at least one pH value between 7 and 11 at which 50 mole percent or more of the groups have a positive charge. Some compounds have covalently attached anionic groups. A group is anionic if, when the compound is in contact with water, there is at least one pH value between 3 and 7 at which 50 mole percent or more of the groups have a negative charge.

Ion exchange resins may be anion exchange resins or cation exchange resins. Anion exchange resins have covalently attached cationic groups. Cation exchange resins have covalently attached anionic groups.

The size distribution of a collection of particles is characterized by passing the collection through a mesh screen having rectangular openings of a specific size. The process of passing the collection through a mesh screen optionally includes mildly vibrating or tapping the sieve that holds the screen.

As used herein, "ambient temperature" is synonymous with "room temperature" and is approximately 23°C.

A drug is a compound that is capable of having a therapeutic effect on a human or other animal. A pharmaceutical composition is a composition that contains one or more drugs. An alkaloid is an organic compound that contains a basic nitrogen atom and that is derived from one or more of the following: pyrrolidine, tropane, pyrrolizidine, piperidine, quinolizidine, indolizidine, pyridine, isoquinoline, oxazole, isoxazole, thiazole, quinazoline, acridine, quinoline, indole, imidazole, purine, β-phenylethylamine, colchicine, muscarine, benzylamine, putrescine, spermidine, spermine, cyclopeptides, diterpenes, and steroids. Alkaloids may be naturally-occurring or synthetic, including compounds made by synthetically altering naturally-occurring compounds. For purposes of the present patent application, amphetamine and substituted amphetamines are considered to be alkaloids derived from β-phenylethylamine, because they are made from ephedrine and/or pseudoephedrine, which are naturally-occurring alkaloids derived from β-phenylethylamine. An opioid is a compound that acts on human opioid receptors to produce effects similar to those produced by morphine. The opioid receptors are G-protein coupled receptors found in the human body.

Ratios are characterized herein as follows. For example, if a ratio is said to be 5:1 or higher, it is meant that the ratio may be 5:1 or 6:1 or 100:1 but may not be 4:1. To state this characterization in a general way, if a ratio is said to be X:1 or higher, then the ratio is Y:1, where Y is greater than or equal to X. Similarly, for example, if a ratio is said to be 2:1 or lower, it is meant that the ratio may be 2:1 or 1:1 or 0.001: 1 but may not be 3:1. To state this characterization in a general way, if a ratio is said to be Z:1 or lower, then the ratio is W:1, where W is less than or equal to Z.

The composition of the present invention contains an ion exchange resin. When anion exchange resins are used, preferred are anion exchange resins with pendant amine groups, which may be primary, secondary, tertiary, or quaternary. Preferred are cation exchange resins; more preferred are ion exchange resins having pendant sulfonic acid groups or carboxyl groups; more preferred are sulfonic acid groups. The sulfonic acid groups or carboxyl groups may be in protonated form or in a salt form.

The ion exchange resin preferably contains polymerized units of one or more acrylic monomer, one or more styrenic monomer, or a mixture thereof. Preferably the total amount of polymerized units of all acrylic monomers and all styrenic monomers is, by weight based on the weight of all polymerized units, 50% or more; more preferably 75% or more; more preferably 90% or more; more preferably 95% or more; more preferably 99% or more.

Two preferred embodiments are considered, herein referred to as "styrenic" embodiments and "acrylic" embodiments. Styrenic embodiments are more preferred.

In acrylic embodiments, the ion exchange resin contains polymerized units of one or more acrylic monomer. In acrylic embodiments, preferably the amount of polymerized units of acrylic monomer is, by weight based on the weight of all polymerized units, 50% or more; more preferably 75% or more; more preferably 80% or more; more preferably 85% or more.

In acrylic embodiments, the ion exchange resin comprises polymerized units of one or more monofunctional acrylic monomer. In acrylic embodiments, preferred are unsubstituted alkyl esters of (meth)acrylic acid and unsubstituted (meth)acrylonitrile; more preferred are methyl acrylate and acrylonitrile. Preferably, the amount of polymerized units of monofunctional acrylic monomer, by weight based on the weight of all polymerized units, is 85% or less; more preferably 80% or less; more preferably 75% or less; more preferably 70% or less.

In styrenic embodiments, preferably, the ion exchange resin contains polymerized units of one or more styrenic monomer. Preferably the amount of polymerized units of styrenic monomer is, by weight based on the weight of all polymerized units, 50% or more; more preferably 75% or more; more preferably 90% or more; more preferably 95% or more; more preferably 99% or more.

In styrenic embodiments, the ion exchange resin comprises polymerized units of one or more monofunctional vinyl monomer. In styrenic embodiments, preferred are styrenic monofunctional vinyl monomer; more preferred are styrene, one or more alkylvinylbenzenes, and mixtures thereof. Among alkylvinylbenzenes, preferred is ethylvinylbenzene. Preferably, the amount of polymerized units of alkylvinylbenzene, by weight based on the weight of all polymerized units, is 0.5% or more; more preferably 1.5% or more; more preferably 3% or more; more preferably 5% or more. Preferably, the amount of polymerized units of alkylvinylbenzene, by weight based on the weight of all polymerized units, is 12% or less; more preferably 10% or less; more preferably 8% or less. Preferably, the amount of polymerized units of styrene, by weight based on the weight of all polymerized units, is 48% or more; more preferably 65% or more; more preferably 72% or more. Preferably, the amount of polymerized units of styrene, by weight based on the weight of all polymerized units, is 97.5% or less; more preferably 93.5% or less; more preferably 88% or less; more preferably 81% or less.

The ion exchange resin comprises polymerized units of one or more multifunctional vinyl monomer. The amount of polymerized units of multifunctional vinyl monomer is, by weight based on the weight of all polymerized units, 14% or more. The amount of polymerized units of multifunctional vinyl monomer is, by weight based on the weight of all polymerized units, 30% or less; more preferably 25% or less; more preferably 20% or less. A preferred multifunctional monomer is divinylbenzene.

The ion exchange resin may be a gel resin or a macroporous resin. Preferred are gel resins.

The size distribution of the collection of particles is characterized by putting the collection in contact with a mesh screen of rectangular openings of a characteristic size. The amount of the collection that passes through the screen and the amount of the collection that is retained on the screen without passing through are noted. Preferably 90% or more of collection of particles by weight passes through a screen having openings of 150 µm. Preferably 90% or more of the particles by weight are retained on a screen having openings of 63 µm. More preferably 90% or more of the particles by weight are retained on a screen having openings of 106 µm.

The collection of particles may be made by any method. Preferably, the particles are first made by a process of aqueous suspension polymerization to make copolymer beads of volume-average diameter of 200 µm or larger. Preferably the monomers used in the aqueous suspension polymerization are styrenic monomers, more preferably styrenic monomers having only atoms of carbon and hydrogen. Preferably the copolymer beads are reacted with appropriate reagents in one or more chemical reactions to attach the desired anionic or cationic groups to make ion exchange resin.

In acrylic embodiments, preferably the copolymer beads are reacted with reactants that include a strong base. Preferred strong bases are alkali metal hydroxides, more preferably sodium hydroxide. Preferably, the strong base reacts with either ester groups or nitrile groups on the copolymer beads to form carboxyl groups.

In styrenic embodiments, preferably, the copolymer beads are reacted with reactants that include sulfuric acid, to attach sulfonic acid groups to the copolymer to make cation exchange resins.

After the desired anionic or cationic groups have been attached, preferably then the ion exchange resin beads are mechanically ground to reduce the volume-average diameter. After grinding, preferably 90% or more of the collection of particles by weight is retained on a mesh screen having openings of 63 µm; more preferably 90% or more of the collection of particles by weight is retained on a mesh screen having openings of 106 µm. After grinding, preferably 90% or more of the collection of particles by weight passes through a mesh screen having openings of 150 µm. In some embodiments, after grinding, a collection of particles may be sorted by passing through various mesh screen, and particles that are undesirably large and particles that are undesirably small may be discarded. It is contemplated that the remaining collection of particles will be a collection of particles of the present invention.

The drug used in the composition of the present invention may be any compound capable of having a therapeutic effect on humans. Preferred drugs have either a cationic group or an anionic group. Preferably, either the drug has a cationic group and the ion exchange resin is a cation exchange resin, or the drug has an anionic group and the ion exchange resin is an anion exchange resin; more preferably, the drug has a cationic group and the ion exchange resin is a cation exchange resin. Preferred drugs have a cationic group that contains a nitrogen atom. Drugs with cationic groups may be in free base form or may be in a salt form.

Preferred drugs are alkaloids. More preferred are alkaloids that are derivatives of one or more of pyrrolidine, tropane, pyrrolizidine, piperidine, quinolizidine, indolizidine, pyridine, isoquinoline, oxazole, isoxazole, thiazole, quinazoline, acridine, quinoline, indole, imidazole, purine, β-phenylethylamine, colchine, muscarine, and benzylamine; more preferred are derivatives of isoquinoline and β-phenylethylamine; more preferred are derivatives of isoquinoline. Among derivatives of β-phenylethylamine, preferred are amphetamine and substituted amphetamines, including bupropion, cathinone, 3,4-methylenedioxymethamphetamine, and methamphetamine. Among derivatives of isoquinoline, preferred are morphine, codeine, and derivatives thereof, including, for example, oxycodone and hydrocodone.

A preferred category of drugs are the opioids.

Preferably, the weight ratio of drug to ion exchange resin is 0.02:1 or higher; more preferably 0.05:1 or higher; more preferably 0.1:1 or higher. Preferably, the weight ratio of drug to ion exchange resin is 1:1 or lower; more preferably 0.8:1 or lower; more preferably 0.6:1 or lower; more preferably 0.4:1 or lower.

Among all embodiments of the present invention, when contemplating the physical relationship between the resin and the drug, two types of preferred embodiments are contemplated, herein labeled "adjacent" embodiments and "ionically bound" embodiments. Preferred are ionically bound embodiments.

In ionically bound embodiments, the drug is ionically bound to the resin. It is preferred that each molecule of the drug be in an ionic complex with a complementary group covalently attached to the ion exchange resin.. In preferred embodiments, the drug has one or more basic groups covalently attached to the drug molecule. The basic group may be, for example, a primary, secondary, or tertiary amine group, which may be in the free base state or in a protonated (i.e., cationic) state. When the drug has one or more basic groups, preferably the ion exchange resin has one or more acidic groups covalently attached to the ion exchange resin. The acidic groups may be, for example, carboxylic acid groups or sulfonic acid groups. The acidic groups may be in the protonated state or in the deprotonated (i.e., anionic) state. Preferably the acidic groups covalently bound to the ion exchange resin form an ionic complex with the basic groups covalently bound to the drug. When the acidic groups on a cation exchange resin have become ionically bound to the basic groups on a drug, the resulting moiety is herein called a "resinate."

In ionically bound embodiments, the drug and the ion exchange resin may be brought together by any method. In a preferred method, the drug is dissolved in water to form a drug solution, and then the drug solution is brought into contact with the ion exchange resin to form a resin slurry. Optionally, water is then removed, for example by filtration, from the resin slurry to form a wet cake. The wet cake is optionally then washed with water and dried.

In adjacent embodiments, the drug is not ionically bound to the resin. In adjacent embodiments, the resin particles are intimately mixed with the drug. For example, the drug may be present as an ingredient in powder particles that are mixed with resin particles and then incorporated into a tablet, a capsule, sprinkles, a gummie, granules, a buccal patch, chewing gum, a lozenge, or a film. For another example, the pharmaceutical composition may be a suspension in which the drug is present either in solution or as an ingredients in particles or droplets that are suspended in the continuous medium and that are separate from the resin particles. It is contemplated that, in adjacent embodiments, the forms and compositions of the various ingredients have been chosen so that, when the pharmaceutical composition is exposed to an aqueous salt solution in an attempt to extract the drug, the aqueous salt solution will facilitate a process in which the drug becomes ionically bound to the resin, thus foiling the attempt to extract the full dose of the drug.

The drug and the ion exchange resin may be present in a composition that contains additional ingredients. Preferably, the drug and ion exchange resin are present in a composition that is appropriate as a dosage form. A dosage form is any composition that can be usefully used for introducing the drug into a human body. Suitable dosage forms are, for example, tablets, capsules, liquids, films, and patches. Tablets may either be designed to be swallowed or may be designed to disintegrate, for example in the mouth. Among tablets, preferred are disintegrating tablets Among liquids, preferred are suspensions, preferably designed to be taken orally. Preferred films are designed to disintegrate, for example in the mouth. Patches are preferably designed to retain intact while the drug diffuses from the patch into the body, for example through skin (transdermal patch) or through mucous membrane (transmucosal patch).

Preferred dosage forms are suspensions, films, tablets, and capsules. More preferred are suspensions, disintegrating tablets, and films; more preferred are suspensions and disintegrating tablets.

When the dosage form is a tablet, preferred additional ingredients include lactose, dibasic calcium phosphate, sucrose, corn starch, microcrystalline cellulose, polyvinyl pyrrolidone, hydroxypropylmethylcellulose, hydroxyethylcellulose, gelatin, polysaccharides, starch, celluloses, hypromellose, glycerine, sorbitol, other sugar alcohols, and combinations thereof.

When the dosage form is a suspension, preferred additional ingredients include one or more of, or any combination of, suspending agents, wetting agents, flocculating agents, thickeners, buffers, osmotic agents, coloring agents, flavoring agents, preservatives, antioxidants, humectants, oils, and external liquid vehicles. Preferred suspending agents include sodium alginate, ethylcellulose, methylcellulose, hydroxyethyl cellulose, hydroxypropyl cellulose, hydroxypropyl methylcellulose, carboxymethyl cellulose, xanthan gum, colloidal silicon dioxide, and mixtures thereof. Preferred wetting agents include nonionic surfactants, hydrophilic colloids (such as acacia, tragacanth, alginates, and guar gum). polyols (such as glycerin, polyethylene glycol, and polypropylene glycol), and polysorbate 80. Preferred buffers include salts of weak acids (such as carbonates, citrates, gluconates, phosphates, and tartrates). Preferred osmotic agents include polyethylene oxide, dextrose, mannitol, sorbitol, sodium chloride, sodium sulfate, and glycerol; more preferred is polyethylene oxide. Preferred preservatives are propylene glycol, disodium EDTA, benzalkonium chloride, benzoic acid, methyl paraben, propyl paraben, and butyl paraben. Preferred antioxidants are ascorbic acid and its derivatives, thiol derivatives, tocopherols, BHA, BHT, sodium bisulfite, and sodium sulfateacetone. Preferred flavoring agents include acacia, anise, benzaldehyde, ginger, glycerin, sarsaparila, spearmint, thyme, sugars (including glucose, fructose, and sucrose), sugar alcohols, sodium cyclamate, sodium saccharin, and aspartame. Preferred coloring agents include FD&C Yellow Number 6, titanium dioxide, brilliant blue, indigo carmine, amaranth, tartarazine, and annatto. Preferred humectants include propylene glycol and glycerol. Preferred oils are vegetable oils.

In an embodiment, it is preferred that the present composition does not contain a sugar alcohol. US 9125948 discloses a composition comprising coated particles of an ion exchange resin loaded with a drug and treated with a sugar alcohol such as sorbitol to reduce swelling of the resin and consequently avoid rupturing the coating. The present inventors have found swelling of the resin particles to be an advantage as swelling increases the viscosity and a highly viscous formulation is more difficult to take up and dispense from a syringe for intravenous injection. Swelling of the resin particles may therefore contribute to the abuse deterrence provided by the composition.

The following are examples of the present invention.

AMBERLITE^{™} IRP69 or AMBERLITE^{™} IRP476 ion exchange resin (6.8 wt% DVB), AMBERJET^{™} 1400 (10 wt% DVB) and AMBERJET^{™} 1600 (15 wt% DVB) were used as starting materials to prepare three resins (labeled Resin-1, Resin-2, and Resin-3). All were gel resins. All had covalently attached sulfonic acid groups. All were copolymers of styrene, divinylbenzene (DVB), and ethylvinylbenzene (EVB). The weight ratio of DVB:EVB was always 63:37. The resins had varying weight % of DVB, as shown below in Table 1. All three were made as relatively large particles by aqueous suspension polymerization, then sulfonated. Each resin was ground two different ways to give two different particle size distributions (63 to 106 µm and 106-150 µm), as shown below in Table 1.

### Example 1: Resinate Samples

Resinate was formed as follows. A 5% by weight solution of hydrocodone bitartrate (available from Noramco) in water was formed. A mixture of resin and solution was formed at a volume ratio of solution to resin of 3.6:1. The mixture was shaken at room temperature for 26 hours. The wet resin was separated by filtration, washed with water, then dried in a fluid bed dryer. The supernatant fluid was filtered, and the concentration of hydrocodone was studied by UV spectrophotometry at 285 nm. Based on the known absorbance of hydrocodone, the absorbance of the solution yielded the concentration of hydrocodone in the supernatant fluid, from which the loading of hydrocodone onto the resin could be deduced, reported below as milligrams of hydrocodone per milligram of ion exchange resin.

In the following table, "size" is characterized by a range, showing a minimum and a maximum. 90% or more by weight of the particles passed through a mesh screen having openings of the maximum size, and 90% or more by weight of the particles were retained on a mesh screen having the minimum size.

**Table 1: Resinate Samples**

| Resinate Number | Resin | wt % DVB | size (µm) | loading (mg/mg) |
|---|---|---|---|---|
| 1* | Resin-1 | 6.8 | 63 to 106 | 0.243 |
| 2* | Resin-1 | 6.8 | 106 to 150 | 0.244 |
| 3* | Resin-2 | 10 | 63 to 106 | 0.243 |
| 4* | Resin-2 | 10 | 106 to 150 | 0.243 |
| 5 | Resin-3 | 15 | 63 to 106 | 0.160 |
| 6 | Resin-3 | 15 | 106 to 150 | 0.134 |

| | | | | |
|---|---|---|---|---|
| * Comparative | | | | |

### Example 2: Extraction Testing

To test the ability of resinate to resist extraction by salt solutions, a dosage form of hydrocodone was used in an amount that had 80 mg of hydrocodone. The dosage form was mixed with either 5 mL or 30 mL of extraction solution. The solutions were 2% NaCl by weight in water and 10% NaCl by weight in water. The mixture was shaken for 30 minutes and filtered. The supernatant liquid was analyzed by ultraviolet absorption at 285 nm. The weight percent of the hydrocodone that was extracted into the extraction solution was reported, based on the total hydrocodone loaded on the resin prior to the extraction. Results were as follows.

**Table 2: Extraction % with NaCl Solutions**

| Resinate | wt % DVB | size (µm) | 5 mL 2% NaCl | 5 mL 10% NaCl | 30 mL 2% NaCl | 30 mL 10% NaCl |
|---|---|---|---|---|---|---|
| 1* | 6.8 | 63 to 106 | 9.33 | 22.51 | 30.52 | 50.10 |
| 2* | 6.8 | 106 to 150 | 8.52 | 20.96 | 26.88 | 37.97 |
| 3* | 10 | 63 to 106 | 12.75 | 23.32 | 30.20 | 37.01 |
| 4* | 10 | 106 to 150 | 11.45 | 20.10 | 25.25 | 28.52 |
| 5 | 15 | 63 to 106 | 9.11 | 9.46 | 12.72 | 12.32 |
| 6 | 15 | 106 to 150 | 7.11 | 8.61 | 10.50 | 10.03 |

| | | | | | | |
|---|---|---|---|---|---|---|
| * Comparative | | | | | | |

When using 5 mL of extraction solution, when comparing results for the same resin, the larger particle-size resinate always allowed less extraction of hydrocodone than the smaller particle-size resinate. Similarly, when using 30 mL of extraction solution, when comparing results for the same resin, the larger particle-size resinate always allowed less extraction of hydrocodone than the smaller particle-size resinate.

When using 5 mL of extraction solution, when comparing resins of the same particle size, the resin having 15% DVB was always showed the lowest amount of extracted hydrocodone. When using 30 mL of extraction solution, when comparing resins of the same particle size, the resin having 15% DVB was always showed the lowest amount of extracted hydrocodone; the comparative resin having 10% DVB showed second-lowest amount of extracted hydrocodone, and the comparative resin having 6.8% DVB showed the highest amount of extracted hydrocodone.

Extraction was also tested with 5 mL of each of the following solutions (by weight % in water): tap water; 10% acetic acid; 10% citric acid; 1% HCl; 40% ethanol. In all cases, the amount of hydrocodone extracted was 5% or less. Additionally, extraction was tested with 30 mL of each of the following solutions (by weight % in water): tap water; 10% acetic acid; 10% citric acid; 1% HCl; 40% ethanol. In all cases, the amount of hydrocodone extracted was 10% or less.

## Claims

1. A pharmaceutical composition comprising a collection of particles, wherein the particles comprise a drug and an ion exchange resin, wherein the ion exchange resin comprises 14% to 30% polymerized units of one or more multifunctional vinyl monomer, by weight based on the dry weight of the ion exchange resin.

2. The composition of claim 1, wherein the ion exchange resin additionally comprises polymerized units of styrene.

3. The composition of claim 1 or 2, wherein the multifunctional vinyl monomer is divinylbenzene.

4. The composition of any one of claims 1-3, wherein the ion exchange resin has sulfonic acid groups.

5. The composition of any one of claims 1-4, wherein the ion exchange resin comprises covalently bound ionic groups, and wherein the drug forms an ionic complex with the ionic groups covalently bound to the ion exchange resin.

6. The composition of any one of claims 1-4, wherein the ion exchange resin comprises no sugar alcohol.

7. The composition of any one of claims 1-6, wherein 90% or more by weight of the particles pass through a mesh screen having openings of 150 µm, and wherein 90% or more by weight of the particles are retained on a mesh screen having openings of 106 µm.

## Patentansprüche

1. Pharmazeutische Zusammensetzung umfassend eine Sammlung von Teilchen, wobei die Teilchen ein Arzneimittel und ein Ionenaustauscherharz umfassen, wobei das Ionenaustauscherharz 14 % bis 30 % polymerisierte Einheiten eines oder mehrerer multifunktioneller Vinylmonomere, gewichtsmäßig auf das Trockengewicht des Ionenaustauscherharzes bezogen, umfasst.

2. Zusammensetzung nach Anspruch 1, wobei das Ionenaustauscherharz zusätzlich polymerisierte Einheiten von Styrol umfasst.

3. Zusammensetzung nach Anspruch 1 oder 2, wobei das multifunktionelle Vinylmonomer Divinylbenzol ist.

4. Zusammensetzung nach einem der Ansprüche 1 bis 3, wobei das Ionenaustauscherharz Sulfonsäuregruppen aufweist.

5. Zusammensetzung nach einem der Ansprüche 1 bis 4, wobei das Ionenaustauscherharz kovalent gebundene ionische Gruppen umfasst und wobei das Arzneimittel einen ionischen Komplex mit den ionischen Gruppen bildet, die kovalent an das Ionenaustauscherharz gebunden sind.

6. Zusammensetzung nach einem der Ansprüche 1 bis 4, wobei das Ionenaustauscherharz keinen Zuckeralkohol umfasst.

7. Zusammensetzung nach einem der Ansprüche 1 bis 6, wobei 90 Gew.-% oder mehr der Teilchen durch ein Maschensieb hindurchgehen, das Öffnungen von 150 µm aufweist und wobei 90 Gew.-% oder mehr der Teilchen auf einem Maschensieb zurückgehalten werden, das Öffnungen von 106 µm aufweist.

## Revendications

1. Composition pharmaceutique comprenant une collection de particules, dans laquelle les particules comprennent un médicament et une résine échangeuse d'ions, et dans laquelle la résine échangeuse d'ions comprend 14 % à 30 % d'unités polymérisées d'un ou plusieurs monomères de vinyle multifonctionnels, en poids rapporté au poids sec de la résine échangeuse d'ions.

2. Composition selon la revendication 1, dans laquelle la résine échangeuse d'ions comprend en outre des unités polymérisées de styrène.

3. Composition selon les revendications 1 ou 2, dans laquelle le monomère de vinyle multifonctionnel est du divinylbenzène.

4. Composition selon l'une quelconque des revendications 1 à 3, dans laquelle la résine échangeuse d'ions comporte des groupes acide sulfonique.

5. Composition selon l'une quelconque des revendications 1 à 4, dans laquelle la résine échangeuse d'ions comprend des groupes ioniques liés par covalence, et dans laquelle le médicament forme un complexe ionique avec les groupes ioniques liés par covalence à la résine échangeuse d'ions.

6. Composition selon l'une quelconque des revendications 1 à 4, dans laquelle la résine échangeuse d'ions ne comprend aucun alcool de sucre.

7. Composition selon l'une quelconque des revendications 1 à 6, dans laquelle 90 % en poids ou plus des particules passent à travers un tamis à mailles ayant des ouvertures de 150 µm, et dans laquelle 90 % en poids ou plus des particules sont retenues sur un tamis à mailles ayant des ouvertures de 106 µm.
